# EUROPEAN PATENT SPECIFICATION

(11) **EP 2 125 056 B1**
(45) Date of publication and mention of the grant of the patent: **18.11.2015**
(21) Application number: 08712718.9
(22) Date of filing: 18.02.2008
(51) Int. Cl.: A61L 27/50, A61F 2/02, A61L 27/30, A61L 27/38

(54) **IMPLANT ASSEMBLY**
IMPLANTATANORDNUNG
ENSEMBLE D'IMPLANT

(30) Priority: 19.02.2007 SE 0700460
(43) Date of publication of application: 02.12.2009
(73) Proprietor: Ticapex AB, 260 40 Viken (SE)
(72) Inventor: BRUCE, Lars, S-260 40 Viken (SE); NILSON, Billy, S-595 30 Mjölby (SE)
(74) Representative: Sjölander, Henrik
(86) International application number: PCT/SE2008/000126
(87) International publication number: WO 2008/103101

(56) References cited:
- EP-A2- 0 747 046
- WO-A1-92/07525
- WO-A1-93/02635
- WO-A1-94/18307
- US-A- 5 344 454
- US-A- 5 344 454
- US-A- 5 807 406
- US-A1- 2006 263 405
- US-B1- 6 372 244

## Description

### TECHNICAL FIELD

The present invention generally relates to an implant assembly, and in particular such an implant assembly comprising a semipermeable membrane or barrier.

### BACKGROUND

It is known in the organ transplantation technique that the human body opposes transplantation of an organ, tissue or even a cluster of cells by rejection phenomena caused by the immune defense of the body. In order to avoid immunosuppressive medicaments, bioartificial implants have been developed which comprise donor tissue or cells, which are to be implanted and a semipermeable barrier or filter, which allows diffusion of nutrients and oxygen from the donee's body to the implanted donor tissue or cell. This barrier should however not allow diffusion of the cells of the donee's immune defense mechanism. In addition and in particular in connection with xenotransplan, it could be advantageous to prevent diffusion of antibodies and components of the complement system of the recipient's body over the semipermeable barrier. The barrier should also of course allow diffusion of the desirable (therapeutic) substances produced by the cells in the implant into the donee's body.

One of the main obstacles in the art for obtaining successful usage of bioartificial implants is the growth of connective tissue in the donee's body around the implant. The connective tissue covers the pores of the semipermeable barrier, effectively inhibiting diffusion of nutrients and oxygen to the cells inside the implant. This will cause hypoxia and cell death.

The document WO 2005/030283 teaches a bioartifical implant, in which the outer surface of the semipermeable barrier is coated with a bioactive metal, such as titanium, in such a way that the coating does not impede the selective diffusion through the membrane. This coating has the advantage of inhibiting the growth of connective tissue (fibrosis) and promoting vessel formation.

US 5,344,454 discloses an implant assembly for holding implanted cells. The implant assembly includes two wall elements and an intermediate sealing element. The wall elements overlie an open area in a sealing member to form a chamber for holding cells for implantation. A sealed region is created between the wall elements and the sealing element to close the chamber. The sealed region is created by means of a sonic weld or by applying an external force to compress the peripheral edges of the wall elements and the sealing element together.

There is though still a need for a bioartificial implant assembly having an implant construction that is highly safe, implying that cells present in the implant assembly cannot unintentionally leak out in the donee body after transplantation. This is particularly important for stem cells and genetically modified cells, which could be transformed into tumor-inducing cells if they escape the implant assembly. There is also a need for an implant assembly having an implant construction designed to minimize the growth of connective tissue.

### SUMMARY

The present invention overcomes these and other drawbacks of the prior art arrangements and meets the needs for an improved bioartificial implant.

It is a general object of the present invention to provide an improved bioartificial implant assembly.

Briefly, the present invention involves a bioartificial implant assembly comprising a frame structure and membranes as diclosed in the appended claims. The frame structure comprises an inwardly protruding support flange and a respective frame portion extending above and below the support flange, respectively. The two membranes have outer circumferences matching the inner circumference of the frame structure. At least one the membranes, preferably both, is semipermeable. One of the membranes is positioned on a first side of the support flange with the other membrane positioned on the second opposite flange side. A sealing is formed at the first frame portion and at the second frame portion. This forms an implant with an enclosed, sealed inner chamber defined by the membranes and the frame structure.

The sealing can be effected by heat pressing the frame portions towards the support flange. Material of the frame portions will penetrate through the semipermeable membrane and attach to the flange material thereby forming effective seals.

Alternatively, foil rings having outer circumferences matching the outer circumference of the frame portions and inner circumferences that are smaller than the outer circumferences of the membranes are positioned on the respective frame portions. The foils rings are sealed, preferably through welding or using an adhesive, to the frame portions but also to the circumferential portions of the membranes.

Instead of utilizing single semipermeable membranes, membrane-comprising laminate structures can be used. In such a case, a sandwich structure comprising a semipermeable membrane, one or more nets of a bioactive metal and optionally one or more weldable rings can be fabricated and handled as a single unit.

### SHORT DESCRIPTION OF THE DRAWINGS

The invention together with further objects and advantages thereof, may best be understood by making reference to the following description taken together with the accompanying drawings, in which:
Fig. 1A is an exploded view of an embodiment of a bioartificial implant assembly according to the present invention;
Fig. 1B illustrates the implant assembly of Fig. 1A before sealing the implant assembly;
Fig. 1C is a cross sectional view of the implant assembly of Fig. 1B;
Fig. 2A illustrates the implant assembly of Fig. 1B after sealing;
Fig. 2B is a cross sectional view of the implant assembly of Fig. 2A;
Fig. 3 is a cross sectional view of another embodiment of a bioartificial implant assembly according to the present invention;
Fig. 4 is an exploded view of a further embodiment of a bioartificial implant assembly according to the present invention;
Fig. 5 is an exploded view of a portion of an embodiment of laminate structure that can be used in the implant assembly according to the present invention;
Fig. 6 is an exploded view of still another embodiment of a bioartificial implant assembly according to the present invention;
Fig. 7A is an exploded view of a further embodiment of a bioartificial implant assembly according to the present invention;
Fig. 7B illustrates the implant assembly of Fig. 7A after sealing the implant assembly;
Fig. 7C is a cross sectional view of the implant assembly of Fig. 7B.
Fig. 8A is a cross sectional view of another laminate structure that can be used in the implant assembly according to the present invention;
Fig. 8B is a cross sectional view of a further laminate structure that can be used in the implant assembly according to the present invention;
Fig. 9A is a cross sectional view of an embodiment of a bioartifical implant assembly according to the present invention housing granulates in its inner chamber;
Fig. 9B is a cross sectional view of an embodiment of a bioartifical implant assembly according to the present invention housing a net in its inner chamber;
Fig. 9C is a cross sectional view of an embodiment of a bioartifical implant assembly according to the present invention housing cells in its inner chamber;
Fig. 10 is a flow diagram illustrating a method of producing a bioartificial implant assembly according to the present invention;
Fig. 11 is a flow diagram illustrating an embodiment of the sealing step of the method illustrated in Fig. 10;
Fig. 12 is a flow diagram illustrating another embodiment of the sealing step of the method illustrated in Fig. 10;
Fig. 13 is a graph illustrating increase in hemoglobin values in mice having received an implant assembly containing EPO-producing cells or control cells;
Fig. 14 is a graph showing percentage of starting value hemoglobin in mice receiving implant assemblies containing EPO-producing cells and having a Biopore membrane or a laminate of a Biopore membrane and titanium net;
Fig. 15 illustrates an implant assembly explanted from a mouse after 24 days. Left panel: removal of the skin tissue, middle panel: vessel formation in the skin tissue facing the implant, and right panel: microscopic view of the skin side of the implant;
Fig. 16 illustrates photographs showing cross-sections of the implant assembly in 100× magnification with implant assemblies without titanium net (upper panels) and with titanium nets (lower panels);
Fig. 17 is a view of the implant assembly with a 0.12 mm net thread size showing the implant wall facing the skin in the left panel and the wall facing the abdomen on the right panel;
Fig. 18 is a view of the implant assembly with a 0.10 mm net thread size showing the implant wall facing the skin in the left panel and the wall facing the abdomen on the right panel;
Fig. 19 is a view of the implant assembly with a 0.10 mm net thread size plus titanium powder coating showing the implant wall facing the skin in the left panel and the wall facing the abdomen on the right panel;
Fig. 20 illustrates a cross section of the wall facing the abdomen of the implant assembly with a 0.12 mm net thread size at 100× magnification; and
Fig. 21 illustrates cross sections of titanium powder coating of the implant assembly with a 0.10 mm net thread size plus titanium coating at 200× magnification (left panel) and 400× magnification (right panel).

### DETAILED DESCRIPTION

Throughout the drawings, the same reference characters will be used for corresponding or similar elements.

The present invention relates to a bioartificial implant assembly as disclosed in the appended claims, based on a semipermeable barrier for allowing selective diffusion of substances over the barrier but also preventing diffusion of cells and possible soluble components, such as antibodies and complement components, of the immune defense. The implant assembly of the present invention has an implant design that reduces the risk for extensive growth of connective tissue around the membrane following implantation or at least reduces the amount of such connective tissue. The implant assembly also has a construction that achieves a highly safe sealing of the interior of the implant, thereby preventing unintentional leakage of, for instance, cells present in the implant interior to the donor body after implantation.

The implant assembly comprises three main constituents: a frame structure and two membranes, of which at least one is semi-permeable. The frame structure comprises an inwardly protruding support flange or shoulder employed as a support structure for the membranes or laminate structures comprising the membranes. A first frame portion of the frame structure extends above the support flange and a second opposite frame portion extends below the support flange as seen from a side view. The first membrane (laminate) is then positioned on a first side of the support flange, while the other membrane (laminate) is positioned on a second opposite side of the support flange. The inner chamber defined by the membranes and the frame structure is then sealed from the outside by a first seal formed at the first frame portion and a second seal formed at the second frame portion.

Herein different embodiments of the bioartificial implant assembly, will be described in more detail in connection with the figures.

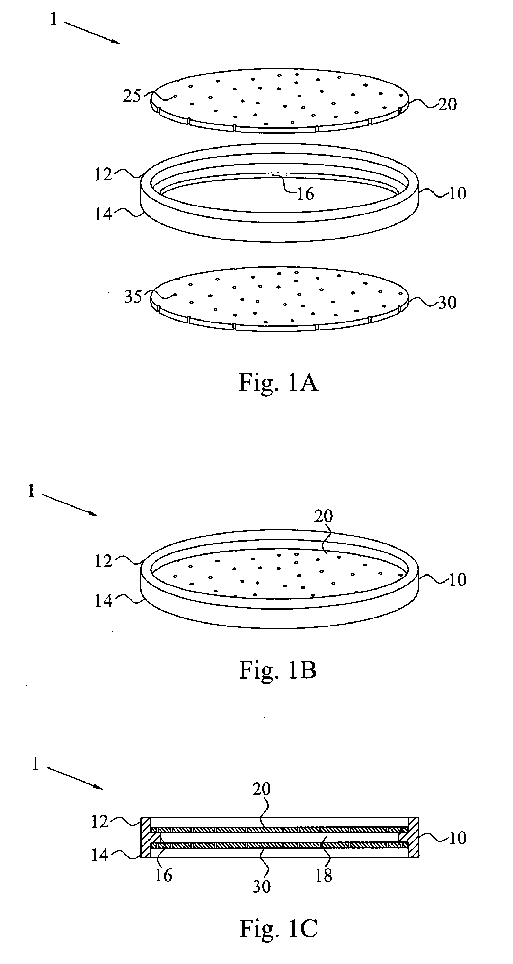

Fig. 1A is a schematic overview of the including elements in an embodiment of the implant assembly 1. The implant assembly 1 comprises a frame structure 10 and at least one semipermeable membrane, barrier or filter 20, 30. The other membrane or barrier 30 is preferably also semipermeable but could alternative be impermeable or have another lower permeability than the first membrane 20. The frame structure 10 comprises an inwardly protruding or extending support flange 16. The support flange 16 is preferably protruding from a central position of the frame backbone 10. This means that a first or upper portion 12 of the frame 10 extends above the flange 16 and a second or lower portion 14 extends below the flange 16.

The frame 10 is preferably made from a biocompatible material. More preferably, the frame 10 is made from a biocompatible polymer material, such as a plastic material. An example of such a material is polyethylene. The general form of the frame 10 could be circular as illustrated in the figure. However, this is merely an illustrative example and the invention is not limited thereto. The invention can thus be used in connection with any general frame form, including also elliptical, triangular, quadratic, or rectangular forms.

In a preferred implementation, the frame 10 is manufacture by molding, such as injection-molding. Other techniques known in the art can be selected to adopt for the particular frame material employed.

The frame thickness could be a few millimeters down to sub-millimeters, for example 1 mm or 0.5 mm. However, the present invention is not limited to any particular frame sizes.

A respective membrane 20, 30 is positioned on either side of and is supported by the flange 16, whereby at least one of the two membranes 20, 30, preferably both membranes 20, 30, constitutes a semipermeable barrier. The semipermeable membranes 20, 30 are designed to, from one side, allow diffusion or prevent diffusion of substances, materials, molecules or cells to the other opposite side. In a preferred implementation, the membranes 20, 30 allow diffusion from one side to the other opposite side of nutrients and oxygen but prevent diffusion of cells and possibly soluble components, in particular cells and soluble components of the immune defense. Furthermore, the membranes 20, 30 are designed to, from the other opposite side, to allow diffusion or prevent diffusion of substances, materials, molecules or cells. In a preferred implementation, the membranes 20, 30 allow diffusion of substances present in the implant assembly 1 or produced by cells in the implant assembly 1 from the other opposite side. In the latter case, the membranes 20, 30 preferably prevent diffusion of any cells in the implant 1 to the outside of the implant assembly 1.

The semipermeable barriers 20, 30 can be made of a polymer material, such as polytetraflouroethylene (PTFE), GoreTex, alginated polymer crosslinked with Ca, carbohydrates, cellulose, plastics, polyvinylchloride (PVC), polycarbonate (PC), polyamides (PA), polyethylene (PE), polypropylene (PP), polyurethane (PU), polyvinylidene fluoride (PVDF), acrylic copolymers, such as polymethylmethacrylate (PMMA) or polyhydroxyethylmethacrylate (PHEMA), polyacetale (POM), polyethersulfone (PES), polysulfone (PS), polyesters, polyetherketone (PEEK), polyetherimide, polyester, polyacrylonitrile (PAN), polyvinylidendifluorid (PVDF), hydrogels. Such membranes 20, 30 are today available at the marked by for instance Millipore Inc, Baxter Inc and GE Healthcare.

Semipermeable membranes 20, 30 are available with different pore size distributions. As is well known in the art, pore sizes for semipermeable membranes are given as nominal pore sizes. In practice, there is a distribution around this average, nominal pore size. For example, typical nominal pore sizes for allotransplantation applications could be 0.45 or 0.22 µm. In the case of xenotransplantation applications, smaller pore size could be useful. The limiting pore size depend on the selective diffusion that is desired, i.e. allowing diffusion of therapeutic material present inside the assembly 1 or being produced inside the assembly 1 to the outside of the membranes 20, 30. Correspondingly, nutrients and oxygen present outside the membranes 20, 30 are preferably allowed to diffuse through the pores 25, 35 into any cells present in the implant assembly 1. However, the pore diameters are preferably not so large to allow cells to pass through the membranes 20, 30.

The size of the membranes 20, 30 is preferably selected so that their outer circumference matches the inner circumference of the frame 10, as is illustrated in Fig. 1B, where one of the membranes 20 has been positioned inside the frame 10 onto the flange 16. The other membrane 30 is likewise positioned on the other side of the flange 16. Fig. 1C is a cross-sectional view of the implant assembly 1 of Fig. 1B. This figure clearly illustrates how the two membranes 20, 30 rest inside the frame 10 on the inwardly protruding flange 16. At this position, the frame 10 and the two membranes 20, 30 define an internal space or chamber 18 that is isolated from the external environment and only accessibly through the pores 25, 35 of the membranes 20, 30. This height of this inner chamber 18 is dictated by the thickness of the support flange 16.

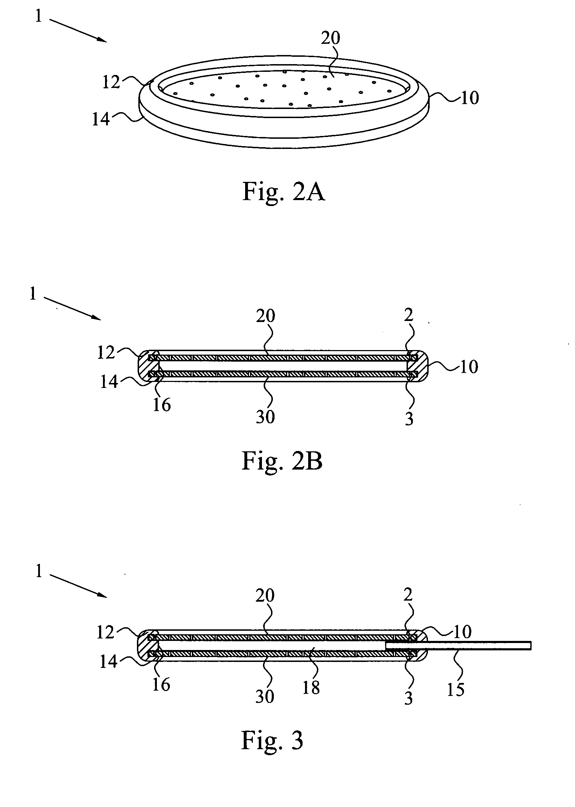

In this embodiment, the implant assembly 1 is closed and sealed by pressing down the extending portions 12, 14 of the frame 10 down towards the flange 16. By also applying heat during the pressing, the extending portions 12, 14 will melt together with the flange 16 to thereby form an effective seal. Fig. 2A schematically illustrates the bioartificial implant 1 after sealing the frame portions 12, 14 onto the flange 16. Fig. 2B is a cross-sectional illustration of Fig. 2A. The seals 2, 3 will lock the membranes 20, 30 to the frame structure 10 as material of the frame 10 will, when applying the heat, flow through the pores of the membrane portions positioned on the flange 16 and contact and melt together with the flange 16. The seals 2, 3 will in this manner be totally secure and prevent any unintentionally leakage of substance between the frame inside and the membranes 20, 30. As is seen best in Fig. 2B, the circumferential portions of the membranes 20, 30 arranged connected to the flange 16 will become embedded in the frame 10.

This implant assembly exhibits several advantages over the prior art implants. Firstly, in the art, the membranes are typically directly welded together or fastened onto each other by applying an adhesive to the circumferential membrane portions. Such a closure may though not be effective enough in clinical applications. Thus, when the implant is transplanted into a host or donee body, the implant will be exposed to strain and external forces due to the movement of body portions. Depending on the actual implantation site, these forces can be large enough to actually break the weld or adhesion between the membranes, allowing content in the implant to leak into the donee body. If the content were cells, these cells will be targeted by the donee's immune defense, effectively killing the cells. The implant will therefore become ineffective. If the cells were, for example, stem cells or genetically modified cells producing a desired therapeutic agent, an undifferentiated form of the stem cells or a modified cell could become a cancer cell in the donee body.

The implant assembly of the invention having a dedicated frame structure supporting the membranes forms an effective seal that will withstand the strain and mechanical forces exerted following transplantation into a donee body, such as an animal body, preferably a mammalian body and more preferably a human body. The frame also provides a semi-rigid backbone that makes the implant less susceptible for the exerted forces.

Another disadvantage of the prior art implants is that, depending on the actual sealing technique employed, the circumferential end portion of the implant can be rough or even sharp. When transplanted, these rough portions will cause a local irritation in the body leading to growth of connective tissue around the implant to encapsulate the irritating implant portions. The connective tissue may, however, form a plug or cover preventing the diffusion of nutrients and oxygen into the inner chamber of the implant and the diffusion of therapeutic substances out from the implant. The cells present in those prior art implants may therefore starve after even a limited transplantation period in the host body due to this connective tissue.

The frame of the invention will after applying the welding (pressure and heat) have smooth, possibly rounded edges as illustrated in Figs. 2A and 2B. There will therefore be no sharp edges that cause local irritation and connective tissue ingrowth.

The bioartificial implant assembly of the invention can be used for various therapeutic or diagnostic applications both in vitro and in vivo. Firstly, the internal space formed by the membranes and the sealed frame can house cells, such as xenogenic cells, allogenic cells or autologous cells. These cells could be genetically modified for producing and releasing a substance or molecule exerting a therapeutic effect when reaching the donee body through the semipermeable membranes. The cells could instead be stem cells or cells derived from stem cells that have the capability of producing an agent of interest. This allows for a controlled and prolonged delivery of the therapeutic agent into the donee body without the risk of the immune defense cells killing the agent-producing cells.

Fig. 3 is a cross-sectional view of another embodiment of an implant assembly 1 of the invention. This implant 1 is equipped with a port 15, illustrated in the form of a tube with an inner channel. This port 15 is arranged in the central portion of the frame 10 and provides a channel into the inner chamber 18. The port 15 can be provided in the frame 10 by inserting the tube 15 into the injection-mold used for forming the frame 10. In an alternative approach, the tube 15 and frame 10 are injection-molded together in a single piece. A further alternative is to first manufacture the frame 10 with a dedicated orifice in its frame backbone and then inserting the tube 15 therein. The tube 15 is then attached to the frame 10 and the orifice is sealed by welding around the lateral tube area in connection with the outer frame wall. However, from a sealing point of view, manufacturing the tube 15 and frame 10 together as a single piece is most preferred, followed by manufacturing the tube 15 separately and inserting the tube 15 into the injection-mold for the frame 10.

The port 15 is preferably centered in the frame structure 10, in other words extends through the center of the frame 10 as illustrated in Fig. 3. This means that the port 15 typically also extends through the support flange 16 in addition to the frame backbone 10. The end of the port 15 positioned in the inner chamber 18 can be aligned with or end closely outside of the inner frame wall or the short end of the support flange 16. Alternatively, the port end can extend some distance into the inner chamber 18 possibly even close up to the opposite inner frame wall or opposite flange end.

The port 15 is in particular suitable for use in connection with a cell application. The implant assembly 10 can then first be manufactured and sealed and then cells, preferably a cell suspension of agent-producing cells and cell medium, are injected using a syringe through the port 15 into the internal space 18. Once the cell suspension has been added to the implant 1, the port 15 can be sealed by welding, by applying heat and pressure or by an adhesive to effectively close the channel. This sealing could be made close to the outer frame wall to form, after the sealing, a smooth outer frame surface. The remaining part of the port 15 is then discarded. However, if the sealing is performed a distance from the frame 10, the internal implant space 18 can be accessed after transplantation by cutting off the seal in the port 15. New cells can then be added to the internal chamber 18 and/or existing cells can be removed therefrom. The port 15 can then once again be sealed. This procedure can be repeated several times depending on the initial length of the port 15.

The implant assembly 1 of the present invention can be equipped with multiple ports 15 if advantageous.

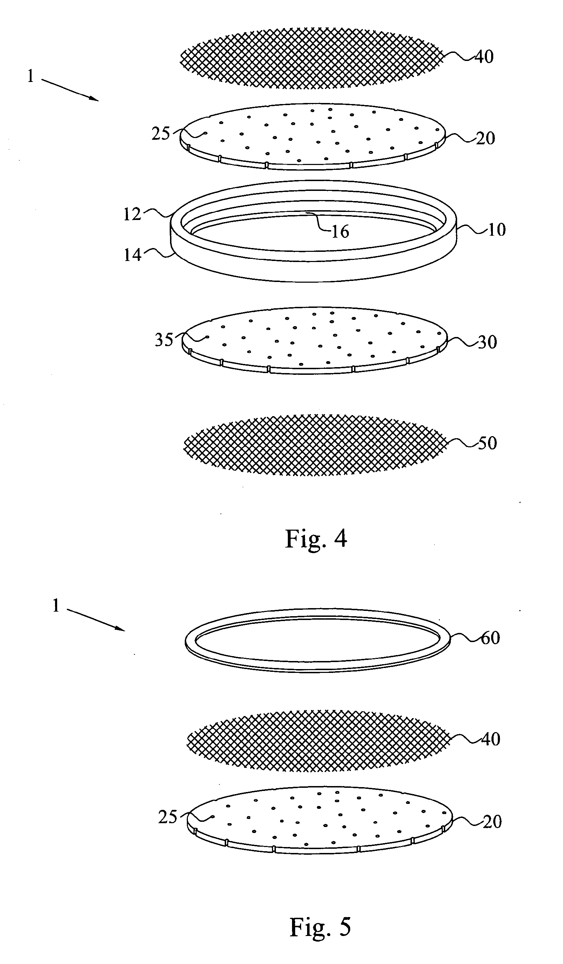

Fig. 4 is a schematic overview of the elements of a further embodiment of an implant assembly 1 of the present invention. In this embodiment, the implant 1 comprises two nets 40, 50 of bioactive metal. These nets 40, 50 have a size similar to the semipermeable membranes 20, 30 and are positioned onto these membranes 20, 30. When sealing the implant 1, both the membranes 20, 30 and the nets 40, 50 will becomes embedded into the frame structure 10.

The bioactive metal is a biocompatible metal that has the capability of attracting tissue and anchoring itself to the tissue. Examples of such materials include titanium, zirconium, tantalum, indium, nickel, aluminum and oxides and alloys thereof, such as titanium dioxide. Other bioactive metals include stainless steel and iridium. Also metals, oxides and alloys comprising chromium, molybdenum, cobalt, tungsten, magnesium, niobium, copper, bismuth, silver, gold, gallium, hafnium, rhenium and zinc could be used. A particular advantageous material is titanium, alloys and oxides thereof.

The nets 40, 50 have several advantageous effects to the implant 1. Firstly, it increases the mechanical strength of the implant 1 and provides stability. This is especially important if the implant 1 is equipped with a port as illustrated in Fig. 3. In such a case, the injection of a cell suspension will exert a pressure towards the membranes 20, 30 causing them to bulge outwards until enough cell medium has diffused through the membrane pores 25, 35. The nets 40, 50 will provide a counter-pressure to this bulging force, preventing the membranes 20, 30 from bulging too much, which may cause damage and even rapture of the membranes 20, 30. The nets 40, 50 also function as a protection to the sensitive semipermeable membranes 20, 30 thereby preventing the rapture of the membranes 20, 30 upon contact with external objects.

Most importantly, the bioactive metal, preferably titanium or an oxide of titanium, of the nets 40, 50 significantly eliminates the fibrosis problem of prior art implants. A possible theory for the prevention of growth of connective tissue around the implant in connection with transplantation into the donee, is that the bioactive metal structure will tightly adhere to and become fixed to surrounding tissue. This adherence reduces the movement of the implant inside the body and thereby reduces the risk of local tissue irritation and fibrosis. In addition, experiments performed indicate that the bioactive metal seems to attract blood vessels in the donee body. The growth of connective tissue close to the net surface will be blocked, and such growth of connective tissue will not have time to start again before the blood vessels grow close to the surface of the bioactive nets 40, 50. This has the further advantage that the blood vessels and growth of blood vessels close to the implant 1 allows nutrients and oxygen from the blood vessels to be effectively transported (diffuse) through the implant unimpeded by connective tissue. The nets 40, 50 therefore lower the risk of hypoxia to cells provided in the internal implant space.

The metal nets 40, 50 are permeable to the substances being able to diffuse through the membranes 20, 30. This means that the openings through the nets 40, 50 have a diameter (size) larger than the pore size of the membranes 20, 30. An example of such a metal net is Tigran titanium net with a pore size of 0.12 mm.

In order to achieve close contact between the nets 40, 50 and the underlying semipermeable membranes 20, 30, a support matrix, such as cell growth support matrix can be provided in the inner chamber enclosed by the membranes 20, 30, which is further discussed herein. The support matrix then pushes the membranes 20, 30 close to the nets 40, 50 and prevents the membranes 20, 30 from bend towards the inner chamber space. If no such support is provided, close contact between net 40, 50 and membrane 20, 30 can be achieved using an adhesive that is provided between the net 40, 50 and the membrane 20, 30. If the adhesive is provided as a layer over the whole membrane surface, it is important that it does not plug the pores in the semipermeable membrane 20, 30. Alternatively, the adhesive could be provided at only a limited portion of the plan membrane surface, thereby leaving the vast majority of membrane pores unaffected by the net-membrane adhesive.

In Fig. 4, the semipermeable membranes 20, 30 and the nets 40, 50 are punched separately from a respective membrane sheet and net sheet. The membranes 20, 30 are first positioned onto the flange 16 followed by the nets 40, 50. An alternatively approach forms a sandwich or laminate structure 100, in which the net 40 is first adhered to the membrane 20 and then the whole structure 100 is positioned on the implant flange. This approach is illustrated in Fig. 5. A carrier foil 60 is first welded to the membrane 20 with the net 40 positioned between the carrier foil 60 and the membrane 20.

In a first approach, a ring 60 is first punched from the carrier foil and the membrane 20, net 40 and punched foil 60 are placed onto each other and aligned as illustrated in Fig. 5. A welding is then applied to the circumferential part of the foil 60, melting the foil 60 through the net 40 and adhering it to the membrane 20.

Alternatively, a hole is first punched through the cover foil 60 where this hole has a circumference slightly less than the circumference of the final laminate structure 100. The net sheet is then placed on the membrane sheet and the punched foil sheet is placed on the net. A welding is applied, causing the protective foil to melt through the net and attach to the membrane. The resulting sandwich structure 100 is punched with a punch having a slightly larger circumference than the punch employed for forming the hole in the cover foil 60. The resulting laminate structure 100 can then be handled in one piece.

Instead of using the net of bioactive metal, a permeable coating of the bioactive metal could be formed from small grains and granulates of the bioactive metal deposited on the semipermeable membranes using some well-known technique, such as atomizing process or thin-film technique, including evaporation or sputtering. Even smaller metal particles, such as metal powder or dust can be "painted" in dry form or in a suspension onto the semipermeable membrane. The coating is preferably substantially continuous to prevent connective tissue form growing on the underlying semipermeable barrier. The coating should also be free from significant knots that could cause growth of connective tissue. It has been found that excellent results (in terms of no clogging of membrane pores, free diffusion of therapeutic substances, nutrients and oxygen through the coating and fibrosis reduction) are achieved using a metal coating thickness of from about 5 nm, preferably about 50 to 250 nm, though also thicker coating such as about or over 1 µm can also be used. For more reference of coating a semipermeable membrane with a bioactive metal reference is made to the patent application WO 2005/030283.

The bioactive coating will react, at transplantation into the donee body, with blood to form a blood clot. This clot works as an adhesive effectively attaching the implant to the surrounding tissue and thereby reducing the risk of inflammation and growth of connective tissue. The porosity of the coat material will have a capillary effect, providing a suction effect towards external tissue. This will enhance the attachment to the tissue.

In an embodiment, a protective membrane can be positioned on the outside of the semipermeable membrane. This protective membrane protects the semipermeable membrane and function as a coating bearing membrane. This means that the coating of the bioactive metal is provided on a first side of the protective membrane, while the second opposite side lies on the membrane. The protective membrane should then be permeability that does not impede with the semipermeability of the semipermeable membrane.

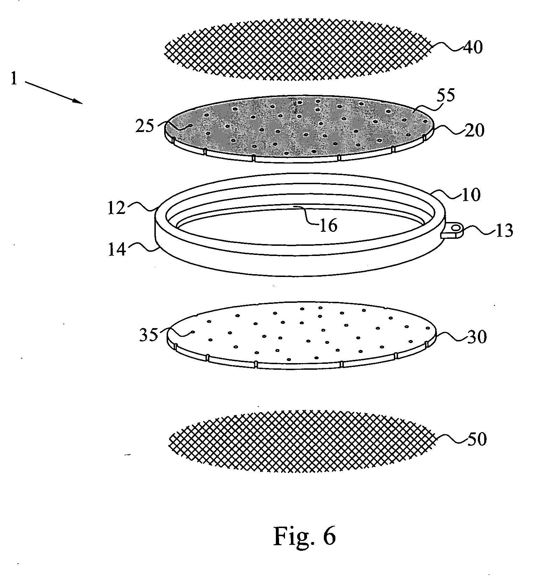

It is actually possible to combine the usage of a net 40, 50 of bioactive metal with a bioactive metal coating 55 on the semipermeable membrane 20, 30, which is illustrated in Fig. 6. In such a case, the implant 1 has the tissue integrating and blood vessel attracting functions of the net 40, 50 and coating 60 and the stabilizing function of the net 40, 50.

The bioactive metal (powder or granulate) coating can alternatively, or preferably in addition, be provided on at least a portion of the outer surface of the frame structure 10. It is indeed possible, during manufacture of the frame 10 to attach titanium oxide powder particles or granulates (or indeed some other bioactive metal) into the outer frame surfaces. This further increases the percentage of the outer implant surface that comprises a bioactive metal. As a consequence, the secure anchoring of the implant into surrounding tissue, the promotion of growth of blood vessels close up to the implant and the suppression of growth of connective tissue are even further boosted by also presenting bioactive metal on the frame surfaces.

As an alternative, or a combination, the final implant assembly of the present invention could, after manufacture, be introduced into a net pouch made of a bioactive metal, such as an oxide of titanium.

Fig. 6 also illustrates another useful element of the frame structure 10, which can be used for securely anchoring the implant assembly 1 to a structure, typically bone structure in the host body. The frame 10 comprises a tissue anchoring structure 13 illustrated in the form of a protruding frame element 13 extending beyond the circumference of the frame 10. This anchoring structure 13 comprises, in this embodiment, a bore through which a bone screw can be entered. Such a screw is then secured (screwed into) a bone of the host body to thereby securely attach the implant 1 to the bone and prevent any significant movement thereof following implantation.

Other forms of tissue anchoring structure could alternatively be employed, including different hooks, barbs and suture sleeves.

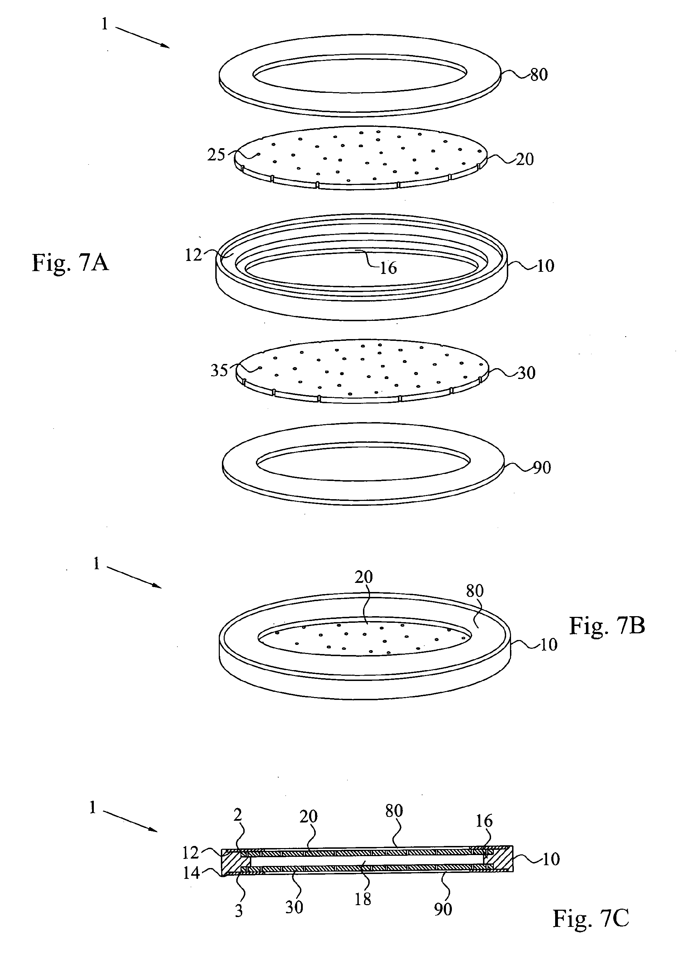

Fig. 7A is an exploded view of another embodiment of the bioartificial implant assembly 1 according to the present invention. As described above, two membranes 20, 30 of which at least one preferably both are semipermeable are positioned on either side of the central support flange 16. In this case, the sealing is not realized by heat pressing the frame portions 12, 14 down towards the flange 16. Instead the frame portions 12, 14 form a sealing surface onto which sealing foil rings 80, 90 are positioned. The foil rings 80, 90 have a respective outer circumference matching the circumference of the frame portions 12, 14. The inner circumference of the rings 80, 90 is though smaller than the outer circumference of the membranes 20, 30. As a consequence, when the rings 80, 90 are positioned on the frame portions 12, 14, the rings 80, 90 will extend slightly over the circumferential portions of the membranes. This is more clearly seen in Fig. 7B and in the cross sectional view of Fig. 7C.

In this case the sealing 2, 3 of the implants are made between the rings 80, 90 and the frame portions 12, 14 and between the rings 80, 90 and the membranes 20, 30 or membrane-containing laminate structures positioned on the flange 16.

A preferred implementation applies a weld between the rings 80, 90 and the frame portions 12, 14 and between the rings 80, 90 and the membranes 20, 30. This weld then forms an effective seal 2, 3 between the frame 10 and the rings 80, 90 and between the rings 80, 90 and the membranes 20, 30. The enclosed interior chamber 18 is fully isolated from the exterior of the implant 1 except through the pores 25, 35 in the membranes 20, 30. An alternative to using welds between ring and frame and ring and membrane is to use and adhesive or a combination of adhesive and welding.

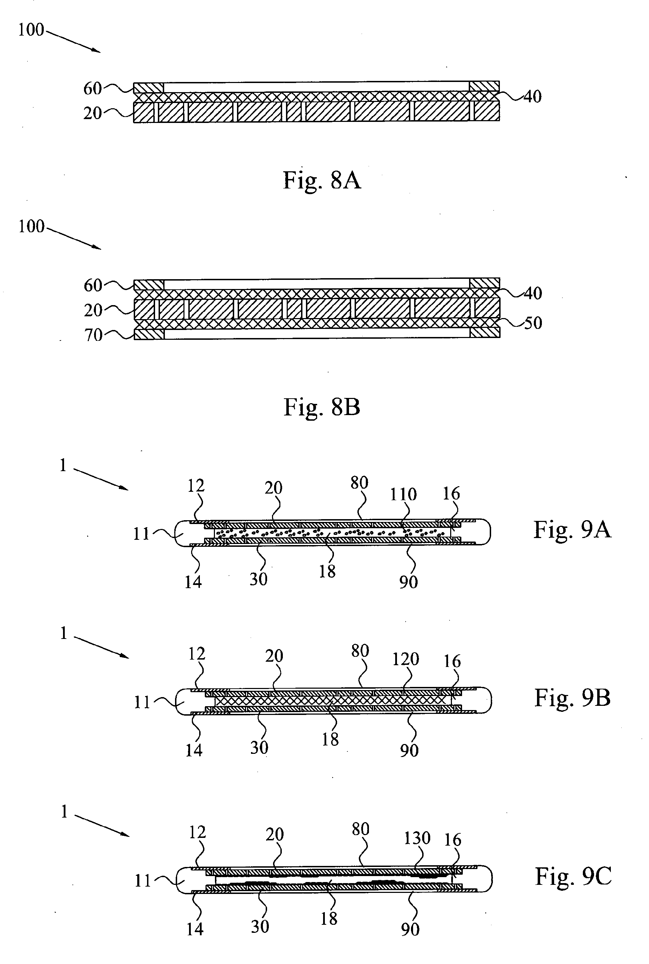

As has been described in the foregoing, the semipermeable membranes of the invention can form part of a laminate structure. Such a structure 100 is illustrated in Fig. 8A. In this laminate embodiment, the structure comprises, in sequential order, a semipermable membrane 20, a net of bioactive metal or metal alloy positioned on the membrane 20 and a cover or weldable ring 60 positioned on the net 40. By welding the ring 60 to the membrane 20, a portion of the ring material will move through the net 40 and attach to the membrane 20. The net 40 therefore becomes fixed to the membrane 20 and the laminate structure 100 can be handled as a single unit.

The laminate structure 100 is positioned on the support flange of the frame structure. With this laminate embodiment, the net 40 can be extended away from the enclosed inner chamber, i.e. facing the exterior of the implant. The net then have the previously described advantageous effects in tissue anchoring, reduction of connective tissue and promoting angiogenesis. Alternatively, the net 40 of the laminate structure 100 faces towards the inner chamber when placed on the support flange. In such a case, the net provides a good cell growth matrix or surface, onto which cells present in the enclosed inner chamber can anchor. This significantly promotes cell survival as the cells then have a favorable surface to attach to and grow on. The net 40 also provides mechanical support and protection to the more sensitive semipermeable membrane 20, in particular when a cell suspension is introduced, such as through the port.

An alternative laminate structure 100 is illustrated in cross sectional view in Fig. 8B. This structure 100 comprises two nets 40, 50 of bioactive metal or metal alloy positioned on either side of the semipermeable membrane 20. A respective weldable ring 60, 70 is positioned outside of the nets 40, 50 and is welded to the membrane 20, thereby anchoring the nets 40, 50 to the membrane 20. This laminate structure 100 presents the bioactive metal nets 40, 50 to both the inner chamber and the implant exterior.

It is anticipated by the present invention that the bioartificial implant assembly of the present invention can have at least one of the semipermeable membranes exchanged for a membrane-containing laminate structure, such as illustrated in Fig. 8A or 8B. This means that the implant can have two single semipermeable membranes, one semipermeable membrane and one membrane-containing laminate structure or two, similar or different, membrane-containing laminate structures, as long as the result falls within the scope of the appended claims.

If the laminate structure illustrated in Fig. 8B is employed or the one in Fig. 8A with the net faced towards the inner chamber, the sealing between the foil ring and the laminate structure is mainly effected between the foil ring and the weldable ring. As a consequence, the respective inner circumferences (diameters in the case of circular implant) of the weldable rings 60, 70 and the foil rings 80, 90 then preferably matches, i.e. being substantially the same.

The weldable rings 60, 70 can be made of weldable materials selected from the ones given for the semipermeable membrane above. A typical example is polyethylene (PE).

In a particular embodiment of the invention, the internal space of the implant can be filled with or partly filled with a cell matrix providing a support for the growth of cells added to the internal chamber. Any such cell matrix available in the art could be used according to the invention. An example is the use of porous titanium grains or granulates 110 or such granulates 110 made of an oxide of titanium as illustrated in Fig. 9A. Titanium granulates 110 produced by the well-known Hunter process or Kroll process are potentially good candidates for cell support. The resulting titanium granulates 110 are highly porous and have a large specific surface area. This specific surface area of the porous titanium granulates 110 is preferably 0.05 m²/g or at least larger than 0.005 m²/g. Suitable titanium granulates 110 for use as cell matrix according to the invention is marked as "Alfa Aesar" (Johnson Matthey), USA, Product No. 042459. An example of granulate size could be 0.1 1 mm to 0.7 mm. The granulates 110 are preferably entered in the enclosed inner chamber 18 before adding one of the membranes 20, 30 or laminate structures to the flange 16. Alternatively and depending on the granulate diameter versus the inner diameter of the port channel, the granulates 110 could be entered in the chamber 18 after sealing and by injection through the port.

The granulates 110 have a further advantageous effect in that the hold the semipermeable membranes 20, 30 in close contact with any net of bioreactive metal that may be provided onto the membranes 20, 30. This in turn reduces the distance between any blood vessels and soft tissue attached to or provided close to the metal net and thereby improves the transport of oxygen and nutrients from the outside into cells present in the cell chamber 18.

Another possible cell matrix providing cell growth support is to use a net 120. This net 120 can, but does not have to be, made of a bioactive metal, preferably a titanium oxide. Other examples include nets made of polymers and plastics. Instead of utilizing nets, different forms of membranes, typically having larger nominal pore size as compared to the semipermeable membranes could be employed. Actually any cell matrix material and design known in the art can be used in connection with the implant assembly 1, including matrices pre-mixed with cells. Fig. 9B illustrates a net 120 provided as cell matrix in the inner chamber 18. The net 120 could free, i.e. not anchored to the frame structure 10 or the membranes 20, 30, or be attached to the implant assembly 1, such as through the frame structure 10. The net 120 could occupy a substantial area of the inner chamber 18 as illustrated in the figure.

It could be possible to use protective membranes in connection with a cell matrix. The protective membranes will then protect the semipermeable membranes from any mechanical ware caused by the cell matrix. In such a case, during manufacture of the implant, a respective protective membrane is first placed onto either side of the flange and then the semipermeable membranes are positioned on the protective membranes. Alternatively, the protective membranes form part of a laminate structure with the membranes and optionally at least one metal net.

The protective membranes should not prevent or negatively effect the diffusion through the semipermeable membranes too much. Thus, their only function is to provide protection to the more sensitive semipermeable membranes.

Fig. 9C illustrates a cross sectional view of the implant assembly 1 having cells 130 attached to the inner chamber walls. These cells 130 are preferably selected, such as genetically modified, for producing and releasing at least one agent of medical interest as is described further herein.

The cross sectional views of Figs. 9A to 9C also illustrate that the outer end of the frame structure 10 is preferably smooth or rounded. This further reduces the risk or irritation and connective tissue growth following implantation. As is seen in the figures, the implant assembly 1 does not present any sharp edges to the outside and instead has a smooth finish.

The teachings of the different embodiment described above and illustrated in the drawings may be combined. For example, the implant assemblies illustrated in Figs. 4, 6, 7 and 9 can be equipped with a port as illustrated in Fig. 3. Furthermore, any of the disclosed assemblies could use an internal cell matrix, such as titanium granulates or net provided in the enclosed implant chamber. Also a laminate structure as illustrated in Fig. 4-6 or 8 can be used in any of the implant assemblies. The tissue anchoring structure of Fig. 6 can be applied to other embodiments of the implant assembly.

The implant assembly of the present invention is particularly suitable as a cell implant, in which the cells are provided in the sealed, enclosed inner chamber. The cells are natural or are genetically engineered for producing and releasing one or more substances that are of medical interest to the exterior of the implant. The substance could be a medical agent that the donee body lacks or has a reduced level of. The implant assembly is though not limited to usage in connection with deficiency diseases. In clear contrast, the cells can produce different types of inhibitors, antagonists, antibodies or agonists that interact with different receptors, proteins and other molecules and cells in the donee body.

Thus, the implant assembly can be used, when filled with one or more cell types, for treating or preventing a disease or medical disorder in an animal body, preferably mammalian body and more preferably human body, where the at least one cell type produces and releases an agent having the capability of treating or preventing the disease or disorder.

In another approach, the bioartificial implant assembly of the invention does not comprise cells as a source of the therapeutic agent. In clear contrast, the agent is provided directly inside the implant chamber, possibly with a suitable excipient, carrier, etc. The agent will then diffuse in a controlled and prolonged way through the pores of the semipermeable membranes. Also a continuous pumping of medicament from an implanted source or external (non-implanted) source through a port into the implant assembly and then out into the recipient body can be used.

Another use of the implant assembly of the invention is a protector or chamber for a diagnostic appliance, such as a sensor head. The sensor is then able to detect the presence of or measure the concentration of a defined substance present in the donee body. This substance is then able to pass through the semipermeable membranes. The implant assembly will protect the sensitive sensor head from mechanical strains. The sensor could in turn be connected to a medical pump, delivering a therapeutic agent to the subject's body in response to the sensor measurements.

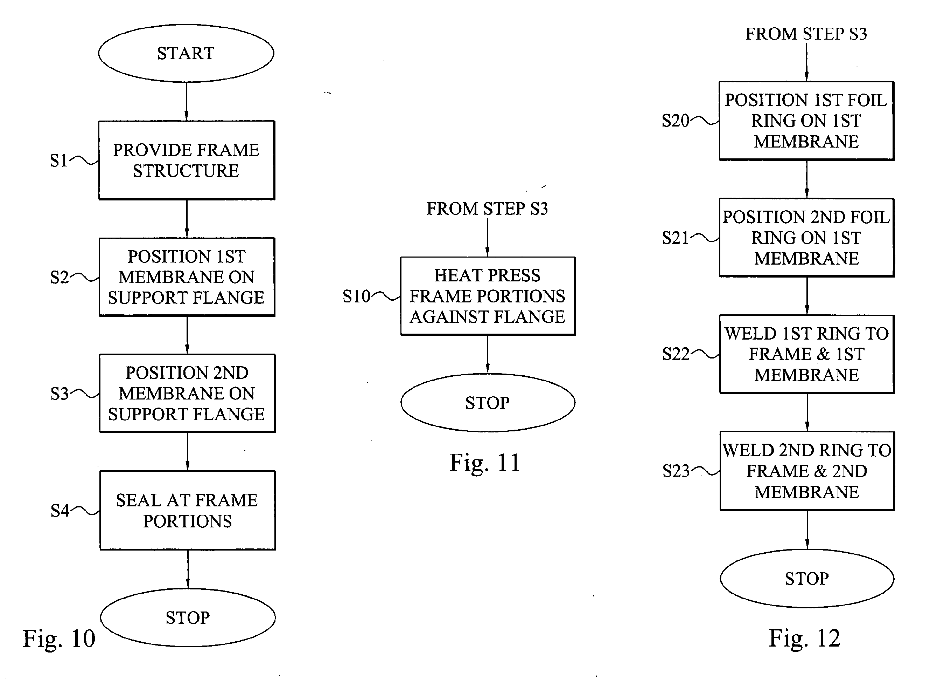

Fig. 10 is a flow diagram illustrating a method of producing a bioartificial implant assembly according to the present invention as disclosed in the appended claims. The method starts in step S1, where the frame structure of the invention having the inwardly protruding support flange and the raised frame portions is provided. This frame structure is manufactured from any of the previously described materials, preferably by injection molding the frame around the port or injection molding the frame and the port in one piece, if a port is used.

A next step S2 positions a first membrane, preferably semipermeable membrane, on a first side of the support flange. For this purpose, the outer circumference (diameter) of the membrane matches (is equal to or close to) the inner circumference (diameter) of the frame structure (10). A second membrane, preferably semipermeable membrane, is likewise positioned on a second opposite side of the support flange. The inner chamber enclosed by the membranes and the frame is sealed in step S4 by forming a first seal in connection with the first frame portion and a second seal in connection with the second frame portion. The method then ends.

The order at which the steps S2 to S4 are performed can differ from what is illustrated in Fig. 10. For instance, the sealing in connection with the first semipermeable membrane can be performed before positioning the second membrane onto the support flange. The sealing of the membranes could alternatively be performed in parallel or in series.

Any material to be provided in the interior of the chamber, such as cell matrix, medicament agent source, including agent-producing cells, can be added prior positioning one of the membranes on the support flange or even after sealing utilizing one or more frame ports.

The correct shape of the semipermeable membrane and optionally the net, protective membrane and rings can be individually formed, such as by punching from larger sheets. The formed membranes, rings and nets can then be placed on the flange or each other to form an optional sandwich structure. Alternatively, sheets are handled together in the form of a prefabricated laminate structure that is positioned onto the flange.

Fig. 11 is a flow diagram illustrating an embodiment of the sealing step of Fig. 10. The method continues from step S3 of Fig. 10. In this embodiment the sealing is effected by heat-pressing (welding) the extending frame-backbone portions down towards the flange to form the implant seal. The material of the frame portions will partly melt through the semipermeable membranes or the membrane-containing laminate structures to connect with and attach to the flange material.

Fig. 12 is a flow diagram illustrating another embodiment of the sealing step of Fig. 10. The method continues from step S3 of Fig. 10. A first foil ring having an outer circumference (diameter) matching (being equal to or close to) the circumference (diameter) of the first frame portion and an inner circumference/diameter being smaller than the outer circumference/diameter of the first membrane is positioned on the first membrane or the first laminate structure. In a next step S21, a corresponding foil ring is positioned on the second membrane or laminate structure. A first seal is effected by welding the first foil ring to the first frame portion and to the first membrane or laminate structure in step S22. A second seal is likewise performed by welding the second foil ring to the second frame portion and to the second membrane or laminate structure in step S23.

The order of the operation steps S20 to S23 can be different from what is illustrated in the figure. For instance, the welding of step S22 can be performed prior to the positioning step S21. Alternatively, steps S20 and S21 are performed in parallel and steps S22 and S23 are performed in parallel.

If a laminate structure is employed instead of single membranes, such a laminate structure can be performed by position a net of a bioactive metal, metal alloy or metal oxide on a first die of the membrane. A weldable ring is positioned on the net and is welded to the membrane to form the laminate structure. In another embodiment, a second net and second weldable ring are welded to the second opposite side of the membrane.

### EXPERIMENT

### (CITED AS AN EXAMPLE AND NOT FORMING PART OF THE INVENTION AS CLAIMED)

Tissue integration was studied using the implant assembly of the present invention containing erythropoietin (EPO) producing cells implanted into mice.

The implant assembly of the present invention was used in connection with Biopore membrane (Millipore, 0.4 µm average pore size) with or without the usage of an outer titanium net (0.12 mm thickness of titanium wire).

These two different types of implant assemblies (with or without net) were filled with allogenic mouse myoblast cells modified to produce EPO. The cells were introduced as a suspension in culture medium through a port of the implant. Thereafter the port was closed by heat-welding. Before implantation, EPO production was tested in culture to assure that the cells were viable.

Male Balb/C mice were anaesthetized using isofluorane and one filled implant assembly was implanted in each mouse. After implantation hemoglobin values were collected and the mice were sacrificed after 24 days. EPO is a hormone that is known to stimulate the production of erythrocytes. Erythrocytes contain hemoglobin (Hgb), which therefore can be used as good indicator on the production of EPO. Vessel formation around the implanted devices was photo documented. One implant assembly per group was cultured after explantation and the release of EPO was followed. Remaining implant assemblies were cross-sectioned and stained using hematoxylineosin.

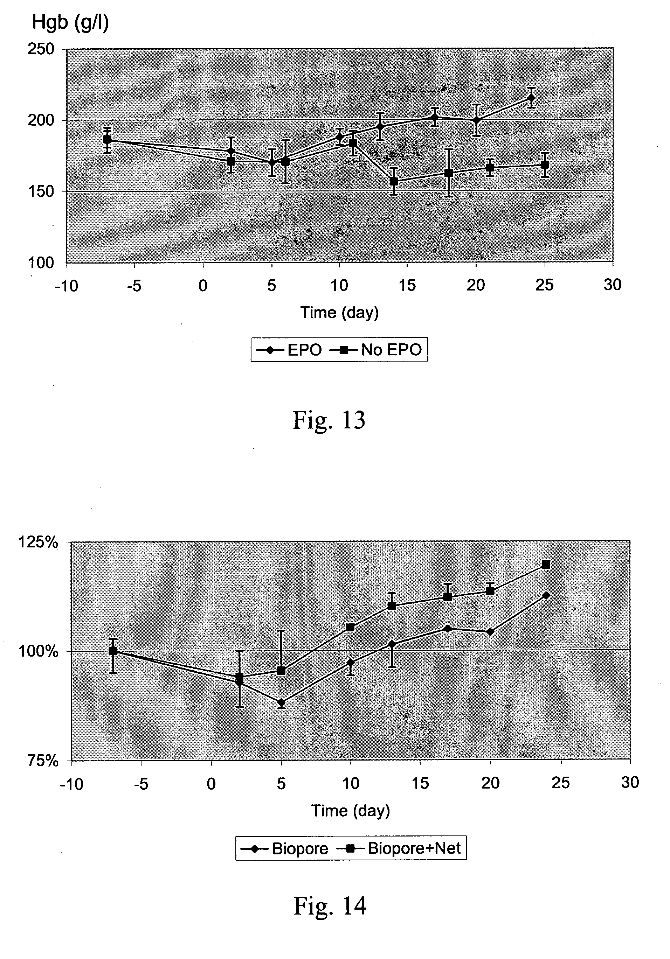

Fig. 13 is a graph showing hemoglobin values in mice that have received the implant assembly containing EPO-producing cells (n=9) or control implant assembly (n=6) without any EPO-producing cells. There is an apparent increase in hemoglobin concentration for those mice having received EPO-producing cells as compared to control mice, thereby illustrating that the enclosed EPO-producing cells are viable in the implant assembly following implantation. The lowered hemoglobin value in the control group between day 11 and day 14 is due to sample collection. Mice in the EPO group were protected from anemia by the released EPO.

Fig. 14 illustrates the results of the comparative study of implant assembly having or not having a titanium net attached on the outside of the Biopore membrane. There is an apparent higher measured hemoglobin concentration for those mice (n=3) having received an implant assembly with titanium net than for those mice (n=3) with an assembly without any net. This indicates that the titanium net is able to improve the cell survival in the implant assembly during implantation.

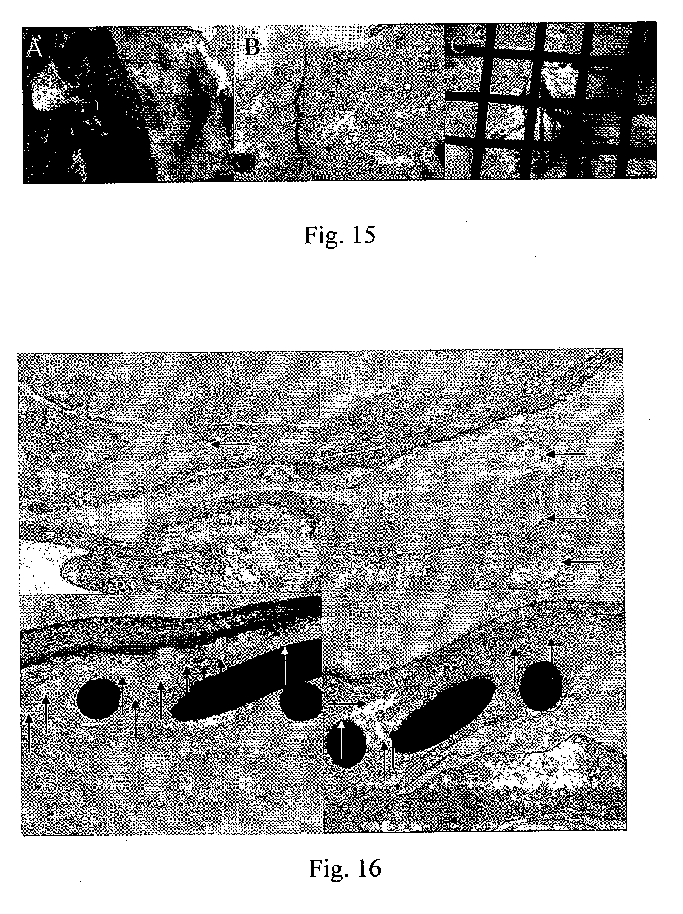

Extensive vessel observation could be seen, see Fig. 15, in the skin tissue of the mice that during the implantation period was in direct contact with the chamber. The left panel of Fig. 15 illustrates the removal of the skin tissue. The vessel formation in the skin tissue facing the implant assembly is clearly seen in the middle panel of Fig. 15. When studied in microscope it was also found that blood vessels had been formed between the titanium net and the Biopore membrane of the implant assembly as is seen in the right panel of Fig. 15.

Fig. 16 provides photographs showing cross-sections of the implant assembly in 100× magnification stained with hematoxylin and eosin. The two upper panels show two different implant assemblies without any titanium net while the lower to panels show two different implants with titanium nets. The black and white arrows in the figure indicate presence of blood vessels. It is seen in the figure that the inclusion of a titanium net causes increased vessel formation around the implant assembly. The arrows are also concentrated to the areas around the threads of the net, further indicating that it is the titanium that promotes the formation of vessels.

Soft-tissue integration of the implant assembly of the invention with different forms of titanium applied to the membrane surface has also been investigated.

Two female Lewis rats received three implant assemblies each. The implant assembly had a laminate structure consisting of an inner non-coated Biopore membrane (Millipore, average pore size of 0.4 µm) onto which a titanium net was positioned, either with a thread size of 0.10 mm or 0.12 mm. The implant assembly with the 0.12 mm thread size had a frame with a thickness of the support flange of about 1.0 mm and was filled with titanium granules (average diameter <0.75 mm) as cell matrix. The implant assembly with thread size of 0.10 mm had a frame with flange thickness of about 0.5 mm. The third assembly type was identical to the implant assembly with thread size of 0.10 mm but was also coated with a layer of titanium oxide powder.

The rats were anaesthetized using isofluorane, approximately 3 % in 400 ml air and 150 ml oxygen per minute. The abdominal skin was opened and devices placed under the fascia connected to the subcutis. The fascia was then closed with a 7-0 suture and the skin was closed with wound clips. After closure of the skin, the animals received Temgesic to achieve pain relief.

The rats were anaesthetized using isofluorane during retrieval. The implant assemblies from one animal were retrieved with as little tissue as possible to visualize vessels on the device. The retrieved implants from the second rat were collected together with surrounding tissue and were placed in 4 % buffered formalin. After retrieval the rats were sacrificed by cutting the heart open.

The retrieved materials were incubated in 4 % formalin for approximately 24 hours before transfer to 70 % ethanol. Plastic embedding, sectioning and staining were performed by HistoCenter AB. The sections were prepared 20 µm thick to avoid loosing metal material. They were then placed on glass slides and stained with hematoxylin and eosin. Digital images of the areas of interest were taken in a light microscope.

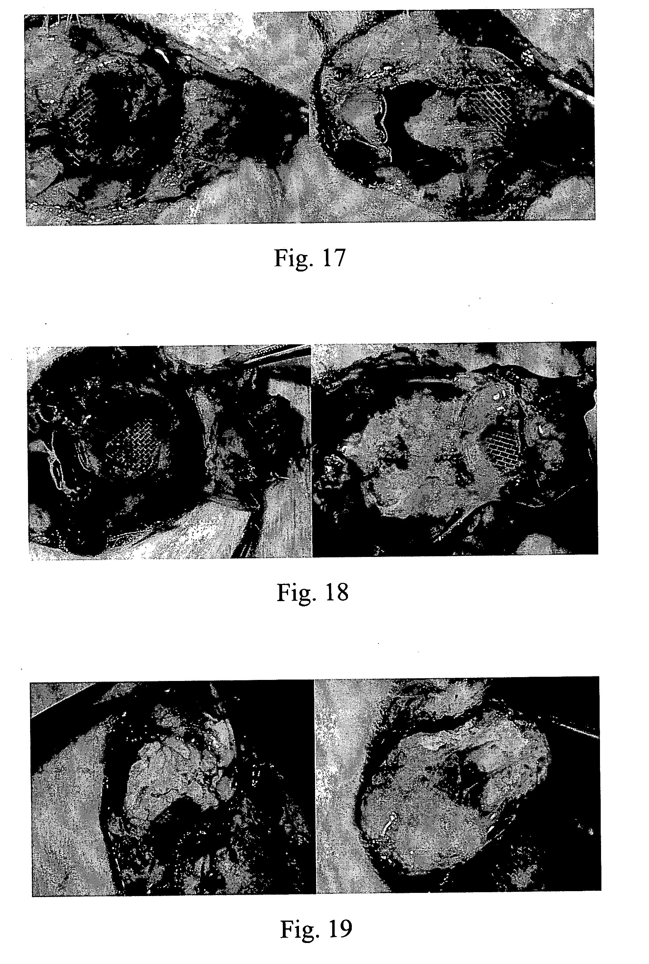

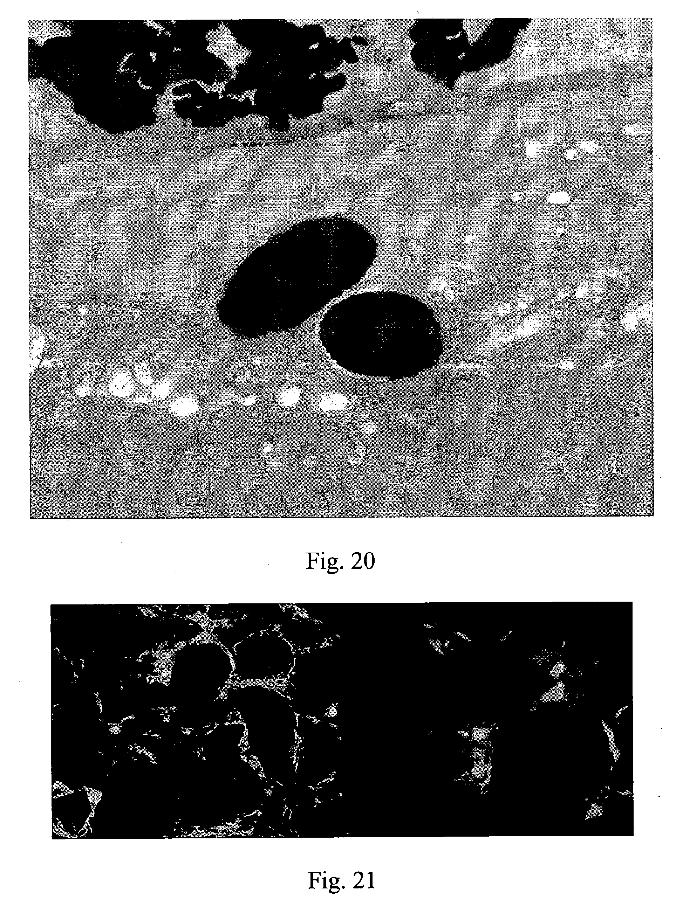

Figs. 17 to 19 illustrates, in left panel, the implant assembly wall facing the skin and, in the right panel, the wall facing the abdomen for the implant assembly with 0.12 mm thread size (Fig. 17), 0.10 mm thread size (Fig. 18) and 0.10 mm thread size + titanium powder coating (Fig. 19). Fig. 20 is the cross-sectional view of the implant wall facing the abdomen at 100× magnification for the 0.12 mm thread size implant. Titanium granulates can be seen above and sectioned threads below the membrane (grey). Fig. 21 illustrate cross-sections of titanium powder coating on the implant with titanium net with 0.10 mm threads and titanium powder at 200x magnification (left panel) and 400x magnification (right panel).

The inclusion of the granulate-based cell matrix improved the close contact between the implant and the tissue, possibly due to that the granulates press the semipermeable membrane close onto the net. Lack of the granulates may cause the membranes to collapse into the enclosed space, thereby obtaining a greater distance between the nets and the membranes.

The application of the titanium powder coating provides a good integration in tissue and also promotes the formation of blood vessels.

It will be understood by a person skilled in the art that various modifications and changes may be made to the present invention without departure from the scope thereof, which is defined by the appended claims.

## Claims

1. A bioartificial implant assembly (1) comprising:
a frame structure (10) comprising an inwardly protruding support flange (16) and a first frame portion (12) extending above said support flange (16);
a first membrane (20) having an outer circumference matching an inner circumference of said frame structure (10) and being positioned on a first side of said support flange (16);
a second membrane (30) having an outer circumference matching said inner circumference of said frame structure (10) and being positioned on a second opposite side of said support flange (16), at least one of said first membrane (20) and said second membrane (30) being a semipermeable membrane; and
a first seal (2) formed at said first frame portion (12), **characterized in that**
said frame structure (10) comprises a second frame portion (14) extending below said support flange (16); and
said bioartificial implant assembly (1) comprises a second seal (3) formed at said second frame portion (14) to form an enclosed inner chamber (18) defined by said first membrane (20), said second membrane (30) and said frame structure (10).

2. The implant according to claim 1, wherein said support flange (16) protrudes from a central position of a backbone of said frame structure (10).

3. The implant according to claim 1 or 2, wherein said frame structure (10) is made from a biocompatible polymer material, such as a biocompatible plastic material.

4. The implant according to any of the claims 1 to 3, wherein said first membrane (20) and said second membrane (30) are semipermeable membranes.

5. The implant according to any of the claims 1 to 4, wherein said first seal (2) is formed by heat pressing said first frame portion (12) against said first side of said support flange (16), and said second seal (3) is formed by heat pressing said second frame portion (14) against said second side of said support flange (16).

6. The implant according to any of the claims 1 to 4, further comprising:
a first foil ring (80) having an outer circumference matching a circumference of said first frame portion (12) and an inner circumference being smaller than said outer circumference of said first membrane (20), said first foil ring (80) being positioned on said first frame portion (12); and
a second foil ring (90) having an outer circumference matching a circumference of said second frame portion (14) and an inner circumference being smaller than said outer circumference of said second membrane (30), said second foil ring (90) being positioned on said second frame portion (14), wherein said first seal (2) is formed between said first foil ring (80) and said first frame portion (12) and between said first foil ring (80) and said first membrane (20) and said second seal (3) is formed between said second foil ring (90) and said second frame portion (14) and between said second foil ring (90) and said second membrane (30).

7. The implant according to claim 6, further comprising a laminate structure (100) comprising, in sequential order, said first membrane (20), a first net (40) of a bioactive metal or an alloy or oxide of said bioactive metal having an outer circumference matching said outer circumference of said first membrane (20) and a first weldable ring (60) having an outer circumference matching said outer circumference of said first membrane (20), said first weldable ring (60) being welded to said first membrane (20), said laminate structure (100) being positioned on said first side of said support flange (16).

8. The implant according to claim 7, wherein said laminate structure (100) comprising, in sequential order, a second weldable ring (70) having an outer circumference matching said outer circumference of said first membrane (20), a second net (50) of a bioactive metal or an alloy or oxide of said bioactive metal having an outer circumference matching said outer circumference of said first membrane (20), said first membrane (20), said first net (40) and said first weldable ring (60), said second weldable ring (70) being welded to said first membrane (20).

9. The implant according to any of the claims 1 to 8, further comprising a chamber port (15) comprising a tube (15) with an inner channel arranged in said frame structure (10) to provide a channel into said enclosed inner chamber (18).

10. The implant according to any of the claims 1 to 9, wherein said enclosed inner chamber (18) comprises granulates (110) of a bioactive metal or an alloy or oxide of said bioactive metal.

11. The implant according to any of the claims 1 to 10, wherein said enclosed inner chamber (18) comprises a net (120) of a bioactive metal or an alloy or oxide of said bioactive metal.

12. The implant according to any of the claims 1 to 11, further comprising a net (40) of a bioactive metal or an alloy or oxide of said bioactive metal having an outer circumference matching said inner circumference of said frame structure (10) and being positioned on a first side of said first membrane (20), said first side being opposite to a second side of said first membrane (20) facing said enclosed inner chamber (18).

13. The implant according to any of the claims 1 to 12, further comprising a permeable coating (60) of a bioactive metal or an alloy or oxide of said bioactive metal provided on a first side of said first membrane (20), said first side being opposite to a second side of said first membrane (20) facing said enclosed inner chamber (18).

14. The implant according to any of the claims 7, 8, 10-13, wherein said bioactive metal or said alloy or oxide of said bioactive metal is selected from titanium, zirconium, tantalum and alloys or oxides thereof, such as titanium dioxide.

15. The implant according to any of the claims 1 to 14, further comprising a tissue anchoring structure (13) connected to and protruding from said frame structure (10).

16. A method of manufacturing a bioartificial implant assembly (1) comprising the steps of:
providing a frame structure (10) comprising an inwardly protruding support flange (16) and a first frame portion (12) extending above said support flange (16);
positioning a first membrane (20) having an outer circumference matching an inner circumference of said frame structure (10) on a first side of said support flange (16);
positioning a second membrane (30) having an outer circumference matching said inner circumference of said frame structure (10) on a second opposite side of said support flange (16), at least one of said first membrane (20) and said second membrane (30) being a semipermeable membrane; and
forming a first seal (2) at said first frame portion (12), **characterized in that**
said frame structure (10) comprises a second frame portion (14) extending below said support flange (16); and
said method further comprising forming a second seal (3) at said second frame portion (14) to form an enclosed inner chamber (18) defined by said first membrane (20), said second membrane (30) and said frame structure (10).

## Patentansprüche

1. Bioartifizielle Implantatanordnung (1), umfassend:
eine Rahmenstruktur (10), die einen nach innen ragenden Trägerbund (16) und einen ersten Rahmenabschnitt (12) umfasst, der über dem Trägerbund (16) verläuft;
eine erste Membran (20) mit einem zum Innenumfang der Rahmenstruktur (10) passenden Außenumfang, die auf einer ersten Seite des Trägerbunds (16) platziert ist;
eine zweite Membran (30) mit einem zum Innenumfang der Rahmenstruktur (10) passenden Außenumfang, die auf einer zweiten gegenüberliegenden Seite des Trägerbunds (16) platziert ist, wobei die erste Membran (20) und/oder die zweite Membran (30) eine semipermeable Membran ist; und
eine erste Dichtung (2), die an dem ersten Rahmenabschnitt (12) ausgebildet ist, **dadurch gekennzeichnet, dass**
die Rahmenstruktur (10) einen zweiten Rahmenabschnitt (14) umfasst, der unter dem Trägerbund (16) verläuft; und
die bioartifizielle Implantatanordnung (1) eine zweite Dichtung (3) umfasst, die an dem zweiten Rahmenabschnitt (14) ausgebildet ist und so eine umschlossene Innenkammer (18) bildet, die von der ersten Membran (20), der zweiten Membran (30) und der Rahmenstruktur (10) definiert ist.

2. Implantat nach Anspruch 1, wobei der Trägerbund (16) aus einer mittigen Stelle eines Gerüsts der Rahmenstruktur (10) ragt.

3. Implantat nach Anspruch 1 oder 2, wobei die Rahmenstruktur (10) aus einem biokompatiblen Polymerwerkstoff wie einem biokompatiblen Kunststoff gefertigt ist.

4. Implantat nach einem der Ansprüche 1 bis 3, wobei die erste Membran (20) und die zweite Membran (30) semipermeable Membranen sind.

5. Implantat nach einem der Ansprüche 1 bis 4, wobei die erste Dichtung (2) durch Heißpressen des ersten Rahmenabschnitts (12) gegen die erste Seite des Trägerbunds (16) ausgebildet wird, und die zweite Dichtung (3) durch Heißpressen des zweiten Rahmenabschnitts (14) gegen die zweite Seite des Trägerbunds (16) ausgebildet wird.

6. Implantat nach einem der Ansprüche 1 bis 4, ferner umfassend:
einen ersten Folienring (80) mit einem Außenumfang, der mit einem Umfang des ersten Rahmenabschnitts (12) zusammenpasst, und einem Innenumfang, der kleiner ist als der Außenumfang der ersten Membran (20), wobei der erste Folienring (80) auf dem ersten Rahmenabschnitt (12) platziert ist; und
einen zweiten Folienring (90) mit einem Außenumfang, der mit einem Umfang des zweiten Rahmenabschnitts (14) zusammenpasst, und einem Innenumfang, der kleiner ist als der Außenumfang der zweiten Membran (30), wobei der zweite Folienring (90) auf dem zweiten Rahmenabschnitt (14) platziert ist, wobei die erste Dichtung (2) zwischen dem ersten Folienring (80) und dem ersten Rahmenabschnitt (12) und zwischen dem ersten Folienring (80) und der ersten Membran (20) ausgebildet ist und die zweite Dichtung (3) zwischen dem zweiten Folienring (90) und dem zweiten Rahmenabschnitt (14) und zwischen dem zweiten Folienring (90) und der zweiten Membran (30) ausgebildet ist.

7. Implantat nach Anspruch 6, ferner umfassend eine Schichtstruktur (100), die nacheinander die erste Membran (20), ein erstes Netz (40) aus einem bioaktiven Metall oder einer Legierung oder einem Oxid des bioaktiven Metalls mit einem Außenumfang, der mit dem Außenumfang der ersten Membran (20) zusammenpasst, sowie einen ersten schweißbaren Ring (60) mit einem Außenumfang umfasst, der mit dem Außenumfang der ersten Membran (20) zusammenpasst, wobei der erste schweißbare Ring (60) an die erste Membran (20) angeschweißt ist, wobei die Schichtstruktur (100) auf der ersten Seite des Trägerbunds (16) platziert ist.

8. Implantat nach Anspruch 7, wobei die Schichtstruktur (100) nacheinander einen zweiten schweißbaren Ring (70) mit einem Außenumfang, der mit dem Außenumfang der ersten Membran (20) zusammenpasst, ein zweites Netz (50) aus einem bioaktiven Metall oder einer Legierung oder einem Oxid des bioaktiven Metalls mit einem Außenumfang, der mit dem Außenumfang der ersten Membran (20) zusammenpasst, die erste Membran (20), das erste Netz (40) und den ersten schweißbaren Ring (60) umfasst, wobei der zweite schweißbare Ring (70) an die erste Membran (20) geschweißt ist.

9. Implantat nach einem der Ansprüche 1 bis 8, ferner umfassend eine eine Röhre (15) mit einem Innenkanal umfassende Kammeröffnung (15), die in der Rahmenstruktur (10) angeordnet ist, damit in die umschlossene Innenkammer (18) hinein ein Kanal vorgesehen ist.

10. Implantat nach einem der Ansprüche 1 bis 9, wobei die umschlossene Innenkammer (18) Granulatpartikel (110) aus einem bioaktiven Metall oder einer Legierung oder einem Oxid des bioaktiven Metalls umfasst.

11. Implantat nach einem der Ansprüche 1 bis 10, wobei die umschlossene Innenkammer (18) ein Netz (120) aus einem bioaktiven Metall oder einer Legierung oder einem Oxid des bioaktiven Metalls umfasst.

12. Implantat nach einem der Ansprüche 1 bis 11, ferner umfassend ein Netz (40) aus einem bioaktiven Metall oder einer Legierung oder einem Oxid des bioaktiven Metalls mit einem Außenumfang, der mit dem Innenumfang der Rahmenstruktur (10) zusammenpasst, das auf einer ersten Seite der ersten Membran (20) platziert ist, wobei die erste Seite einer zweiten Seite der ersten Membran (20) gegenüberliegt, die der umschlossenen Innenkammer (18) zugewandt ist.

13. Implantat nach einem der Ansprüche 1 bis 12, ferner umfassend eine durchlässige Beschichtung (60) aus einem bioaktiven Metall oder einer Legierung oder einem Oxid des bioaktiven Metalls, die auf einer ersten Seite der ersten Membran (20) vorgesehen ist, wobei die erste Seite einer zweiten Seite der ersten Membran (20) gegenüberliegt, die der umschlossenen Innenkammer (18) zugewandt ist.

14. Implantat nach einem der Ansprüche 7, 8, 10 bis 13, wobei das bioaktive Metall oder die Legierung oder das Oxid des bioaktiven Metalls aus Titan, Zirconium, Tantal und Legierungen oder Oxiden davon wie Titandioxid ausgewählt ist.

15. Implantat nach einem der Ansprüche 1 bis 14, ferner umfassend eine Gewebeverankerungsstruktur (13), die mit der Rahmenstruktur (10) verbunden ist und aus ihr herausragt.

16. Verfahren zur Herstellung einer bioartifiziellen Implantatanordnung (1), das folgende Schritte umfasst:
Bereitstellen einer Rahmenstruktur (10), die einen nach innen ragenden Trägerbund (16) und einen ersten Rahmenabschnitt (12) umfasst, der über dem Trägerbund (16) verläuft;
Platzieren einer ersten Membran (20) mit einem zum Innenumfang der Rahmenstruktur (10) passenden Außenumfang auf einer ersten Seite des Trägerbunds (16);
Platzieren einer zweiten Membran (30) mit einem zum Innenumfang der Rahmenstruktur (10) passenden Außenumfang auf einer zweiten gegenüberliegenden Seite des Trägerbunds (16), wobei die erste Membran (20) und/oder die zweite Membran (30) eine semipermeable Membran ist; und
Formen einer ersten Dichtung (2) an dem ersten Rahmenabschnitt (12), **dadurch gekennzeichnet, dass**
die Rahmenstruktur (10) einen zweiten Rahmenabschnitt (14) umfasst, der unter dem Trägerbund (16) verläuft; und
wobei das Verfahren ferner das Formen einer zweiten Dichtung (3) an dem zweiten Rahmenabschnitt (14) zur Bildung einer umschlossenen Innenkammer (18) umfasst, die durch die erste Membran (20), die zweite Membran (30) und die Rahmenstruktur (10) definiert ist.

## Revendications

1. Ensemble d'implant bioartificiel (1), comprenant :
une structure de cadre (10) comprenant un rebord de support (16) dépassant vers l'intérieur et une première partie de cadre (12) s'étendant au-dessus dudit rebord de support (16) ;
une première membrane (20) présentant une circonférence extérieure correspondant à une circonférence intérieure de ladite structure de cadre (10) et qui est positionnée sur un premier côté dudit rebord de support (16) ;
une seconde membrane (30) présentant une circonférence extérieure correspondant à ladite circonférence intérieure de ladite structure de cadre (10) et qui est positionnée sur un second côté opposé dudit rebord de support (16), ladite première membrane (20) et/ou ladite seconde membrane (30) étant une membrane semi-perméable ; et
un premier joint d'étanchéité (2) formé sur ladite première partie de cadre (12), **caractérisé en ce que**
ladite structure de cadre (10) comprend une seconde partie de cadre (14) s'étendant sous ledit rebord de support (16) ; et
ledit ensemble d'implant bioartificiel (1) comprend un second joint d'étanchéité (3) formé sur ladite seconde partie de cadre (14) pour former une chambre intérieure enfermée (18) définie par ladite première membrane (20), ladite seconde membrane (30) et ladite structure de cadre (10).

2. Implant selon la revendication 1, dans lequel ledit rebord de support (16) dépasse d'une position centrale d'une ossature de ladite structure de cadre (10).

3. Implant selon la revendication 1 ou 2, dans lequel ladite structure de cadre (10) est faite à partir d'un matériau polymère biocompatible, tel qu'une matière plastique biocompatible.

4. Implant selon l'une quelconque des revendications 1 à 3, dans lequel ladite première membrane (20) et ladite seconde membrane (30) sont des membranes semi-perméables.

5. Implant selon l'une quelconque des revendications 1 à 4, dans lequel ledit premier joint d'étanchéité (2) est formé en pressant à chaud ladite première partie de cadre (12) contre ledit premier côté dudit rebord de support (16), et ledit second joint d'étanchéité (3) est formé en pressant à chaud ladite seconde partie de cadre (14) contre ledit second côté dudit rebord de support (16).

6. Implant selon l'une quelconque des revendications 1 à 4, comprenant en outre :
un premier anneau de feuille (80) présentant une circonférence extérieure correspondant à une circonférence de ladite première partie de cadre (12) et une circonférence intérieure qui est inférieure à ladite circonférence extérieure de ladite première membrane (20), ledit premier anneau de feuille (80) étant positionné sur ladite première partie de cadre (12) ; et
un second anneau de feuille (90) présentant une circonférence extérieure correspondant à une circonférence de ladite seconde partie de cadre (14) et une circonférence intérieure qui est inférieure à ladite circonférence extérieure de ladite seconde membrane (30), ledit second anneau de feuille (90) étant positionné sur ladite seconde partie de cadre (14), ledit premier joint d'étanchéité (2) étant formé entre ledit premier anneau de feuille (80) et ladite première partie de cadre (12) et entre ledit premier anneau de feuille (80) et ladite première membrane (20), et ledit second joint d'étanchéité (3) est formé entre ledit second anneau de feuille (90) et ladite seconde partie de cadre (14) et entre ledit second anneau de feuille (90) et ladite seconde membrane (30).

7. Implant selon la revendication 6, comprenant en outre une structure stratifiée (100) comprenant, dans l'ordre, ladite première membrane (20), un premier filet (40) d'un métal bioactif ou d'un alliage ou un oxyde dudit métal bioactif présentant une circonférence extérieure correspondant à ladite circonférence extérieure de ladite première membrane (20) et un premier anneau soudable (60) présentant une circonférence extérieure correspondant à ladite circonférence extérieure de ladite première membrane (20), ledit premier anneau soudable (60) étant soudé à ladite première membrane (20), ladite structure stratifiée (100) étant positionnée sur ledit premier côté dudit rebord de support (16).

8. Implant selon la revendication 7, dans lequel ladite structure stratifiée (100) comprend, dans l'ordre, un second anneau soudable (70) présentant une circonférence extérieure correspondant à ladite circonférence extérieure de ladite première membrane (20), un second filet (50) d'un métal bioactif ou d'un alliage ou un oxyde dudit métal bioactif présentant une circonférence extérieure correspondant à ladite circonférence extérieure de ladite première membrane (20), ladite première membrane (20), ledit premier filet (40) et ledit premier anneau soudable (60), ledit second anneau soudable (70) étant soudé à ladite première membrane (20).

9. Implant selon l'une quelconque des revendications 1 à 8, comprenant en outre un orifice de chambre (15) comprenant un tube (15) avec un canal intérieur agencé dans ladite structure de cadre (10) afin de réaliser un canal menant dans ladite chambre intérieure enfermée (18).

10. Implant selon l'une quelconque des revendications 1 à 9, dans lequel ladite chambre intérieure enfermée (18) comprend des granulés (110) d'un métal bioactif ou d'un alliage ou un oxyde dudit métal bioactif.

11. Implant selon l'une quelconque des revendications 1 à 10, dans lequel ladite chambre intérieure enfermée (18) comprend un filet (120) d'un métal bioactif ou d'un alliage ou un oxyde dudit métal bioactif.

12. Implant selon l'une quelconque des revendications 1 à 11, comprenant en outre un filet (40) d'un métal bioactif ou d'un alliage ou un oxyde dudit métal bioactif, présentant une circonférence extérieure correspondant à ladite circonférence intérieure de ladite structure de cadre (10) et qui est positionnée sur un premier côté de ladite première membrane (20), ledit premier côté étant opposé à un second côté de ladite première membrane (20) qui fait face à ladite chambre intérieure enfermée (18).

13. Implant selon l'une quelconque des revendications 1 à 12, comprenant en outre un revêtement perméable (60) d'un métal bioactif ou d'un alliage ou un oxyde dudit métal bioactif situé sur un premier côté de ladite première membrane (20), ledit premier côté étant opposé à un second côté de ladite première membrane (20) qui fait face à ladite chambre intérieure enfermée (18).

14. Implant selon l'une quelconque des revendications 7, 8, 10 à 13, dans lequel ledit métal bioactif ou ledit alliage ou oxyde dudit métal bioactif est choisi parmi le titane, le zirconium, le tantale et des alliages ou oxydes de ceux-ci, tels que le dioxyde de titane.

15. Implant selon l'une quelconque des revendications 1 à 14, comprenant en outre une structure d'ancrage de tissu (13) relié à ladite structure de cadre (10) et dépassant de celle-ci.

16. Procédé de fabrication d'un ensemble d'implant bioartificiel (1), comprenant les étapes consistant à :
mettre à disposition une structure de cadre (10) comprenant un rebord de support (16) dépassant vers l'intérieur et une première partie de cadre (12) s'étendant au-dessus dudit rebord de support (16) ;
positionner une première membrane (20) présentant une circonférence extérieure correspondant à une circonférence intérieure de ladite structure de cadre (10) sur un premier côté dudit rebord de support (16) ;
positionner une seconde membrane (30) présentant une circonférence extérieure correspondant à ladite circonférence intérieure de ladite structure de cadre (10) sur un second côté opposé dudit rebord de support (16), ladite première membrane (20) et/ou ladite seconde membrane (30) étant une membrane semi-perméable ; et
former un premier joint d'étanchéité (2) sur ladite première partie de cadre (12), **caractérisé en ce que**
ladite structure de cadre (10) comprend une seconde partie de cadre (14) s'étendant sous ledit rebord de support (16) ; et
ledit procédé comprenant en outre le fait de former un second joint d'étanchéité (3) sur ladite seconde partie de cadre (14) pour former une chambre intérieure enfermée (18) définie par ladite première membrane (20), ladite seconde membrane (30) et ladite structure de cadre (10).
